# EUROPÄISCHE PATENTANMELDUNG

(11) **EP 0 564 396 A1**
(43) Veröffentlichungstag der Anmeldung: **06.10.1993**
(21) Anmeldenummer: 93810128.4
(22) Anmeldetag: 24.02.1993
(51) Int. Cl.: B08B 7/00, D06F 43/00

(54) **Verfahren und Vorrichtung zur Reinigung und Keimverminderung von textilen medizinischen Implantaten**

(30) Priorität: 01.04.1992 EP 92810244
(71) Anmelder: SULZER Medizinaltechnik AG, CH-8404 Winterthur (CH)
(72) Erfinder: Steininger, Roland, Dr., CH-8400 Winterthur (CH); Zehnder, Beat, Dr., CH-8600 Dübendorf (CH); Quack, Hans, Dr., CH-8330 Pfäffikon (CH)
(74) Vertreter: Hammer, Bruno, Dr.

(57) **Zusammenfassung**

Die Erfindung zeigt eine Hochdruck-Waschkammer (1) in der ein Waschgut, das aus textilen medizinischen Implantaten besteht, von der Atmosphäre isoliert eingeschlossen ist. Das textile medizinische Implantat (2) wird von einem überkritischen oder als flüssiges Fluid ausgebildeten Waschmedium (40) für eine bestimmte Waschzeit durchströmt, wobei das Fluid bei Umgebungsbedingungen aus mindestens einem nicht-toxischen als Hauptwaschmittel wirkenden Gas (38) besteht, dem nach Bedarf weitere Gase und/oder flüssige Lösungsmittel (44) beigemischt werden können. Das in Vorratsbehältern (14, 23, 24) in flüssiger Form gelagerte Waschmedium (40) wird durch Pumpen (16, 36, 37) auf einen so hohen Druck gebracht, dass es nach dem Zusammenmischen die Waschkammer (1) bei der vorgesehenen Waschtemperatur als überkritisches oder flüssiges Fluid durchströmt. Dabei sind Temperatur und Druck des Waschmediums (40) entsprechend einem vorgegebenen Programm regelbar, um den keimvermindernden überkritischen Zustand mit seiner Temperatur einer maximal zulässigen Temperatur des textilen medizinischen Implantats (2) anzupassen.

## Beschreibung

Die Erfindung handelt von einem Verfahren und einer Vorrichtung zur Reinigung und Keimverminderung von textilen medizinischen Implantaten.

Textile medizinische Implantate, die in Reinraumtechnik verarbeitet werden müssen, erfordern eine gründliche Reinigungsprozedur und viel Aufwand, um die Keimzahlen niedrig zu halten. Bisher war es üblich solches Waschgut in Industriewaschmaschinen oder in abgeänderten handelsüblichen Waschmaschinen mit wässrigen Lösungen, denen Waschmittel oder Lösungsmittel zugesetzt sind, zu reinigen.

Eine andere Methode besteht darin, textile medizinische Implantate nach einem Programm in Behälter mit verschiedenen wässrigen Lösungen nacheinander einzubringen, um die Verschmutzungen unter Zuhilfenahme der Waschmittelzusätzche zu isolieren oder zu lösen und auszuspülen. Dabei können die eingesetzten Waschmedien ihrerseits (auch toxische) Rückstände auf dem textilen medizinischen Implantat zurücklassen, die durch zusätzliches mehrmaliges Spülen eliminiert werden müssen. Bei beiden Waschmethoden geht ein solches Waschprogramm über mehrere Stunden und verlangt über diese Zeit klinische Sauberkeit für die beteiligten Apparate und Maschinen, für die Atmosphäre und für das Bedienungspersonal.

Ein Nachteil dieser Anordnung besteht darin, dass die Anzahl der Keime in nützlicher Zeit ungenügend reduziert wird und dass sämtliche verbleibende Keime in einer Sterilisations- und Verpackungsstation mit dem Verpacken des textilen medizinischen Implantats abgetötet werden müssen.

In der Patentanmeldung WO 90/06189 ist ein allgemeines Waschverfahren mit einem überkritischen Fluid beschrieben, bei dem die Verschmutzungen in Lösung gehen, um mit dem Fluid abgezogen zu werden. Theoretisch wäre eine Sterilisation während dem Waschverfahren möglich, wenn stark toxische Medien wie Ozon als Waschmedien bei entsprechend hohen Temperaturen verwendet würden. Praktisch besteht hierbei die Gefahr von Rückständen auf den textilen medizinischen Implantaten und durch die Verwendung eine Gefährdung des Bedienungspersonals.

Aufgabe der Erfindung ist es, in einem kurzen Reinigungsverfahren mit einer zugehörigen Vorrichtung während dem Waschprozess die Anzahl der Keime wesentlich herabzusetzen.

Gemäss der Erfindung wird die Aufgabe mit den Merkmalen der unabhängigen Patentansprüchen 1 und 11 gelöst.

Die Vorteile der Erfindung liegen darin, dass auch bei Temperaturen, die weit unter der Dampfsterilisationstemperatur liegen und die für die mechanischen Eigenschaften des textilen medizinischen Implantats ungefährlich sind, die Anzahl der Keime um einen Faktor von mehreren Zehnerpotenzen reduziert wird.

Es ist ein Ziel der Erfindung, dass in der Endsterilisation nicht zu viele Keime anfallen, um den Sterilisationsvorgang einfach zu halten. Es ist ein weiteres Ziel, die Zahl der Keime vor der Endsterilisation niedrig zu halten, damit die toxische Wirkung der toten Keime niedrig gehalten wird. Bakterielle Toxine werden nicht mit dem Tod der Zelle inaktiviert. Bei gewissen Sorten liegt die tödliche Dosis für Menschen in der Grössenordnung von einem Mikrogramm. Gefährlich sind die hitzebeständigen Pyrogene. Pyrogen wird von zahlreichen gramnegativen Bakterien wie z.B. Pseudomonaden, Salmonellen und E. Coli produziert. Es ist dort bis zu etwa 90 % an die Zellwand gebunden. Während die gramnegativen Bakterien bereits bei Temperaturen von 70°C nach 30 Min. abgetötet werden, sind die aus ihnen freiwerdenden Pyrogene relativ hitzestabil und werden erst ab Temperaturen über 200°C abgetötet. Bruchteile eines Mikrogramms verursachen beim Kaninchen, intravenös verabreicht, innerhalb von 30 bis 60 Min. eine Temperaturerhöhung von 0.8 bis 1.5°C.

Pyrogene können durch Prozesswasser oder Rohstoffe eingeschleppt werden. In dieser Hinsicht ist es sicher ein Vorteil, dass der Waschprozess ohne Wasser durchgeführt wird.

Ausserdem ist günstig, dass lösliche Verschmutzungen durch die beschriebene Anordnung bei dem Waschmedium schnell in Lösung gehen und dass der Verbrauch an Waschmedium gering ist. Ein weiterer Vorteil besteht darin, dass das eingesetzte Waschmedium keine Rückstände auf dem Waschgut zurücklässt und dass weitere Spülungen entfallen.

Die Unteransprüche 2 bis 10 und 12 bis 16 stellen vorteilhafte Weiterbildungen der Erfindung dar. Im folgenden ist die Erfindung anhand eines Ausführungsbeispiels näher beschrieben. Es zeigt:

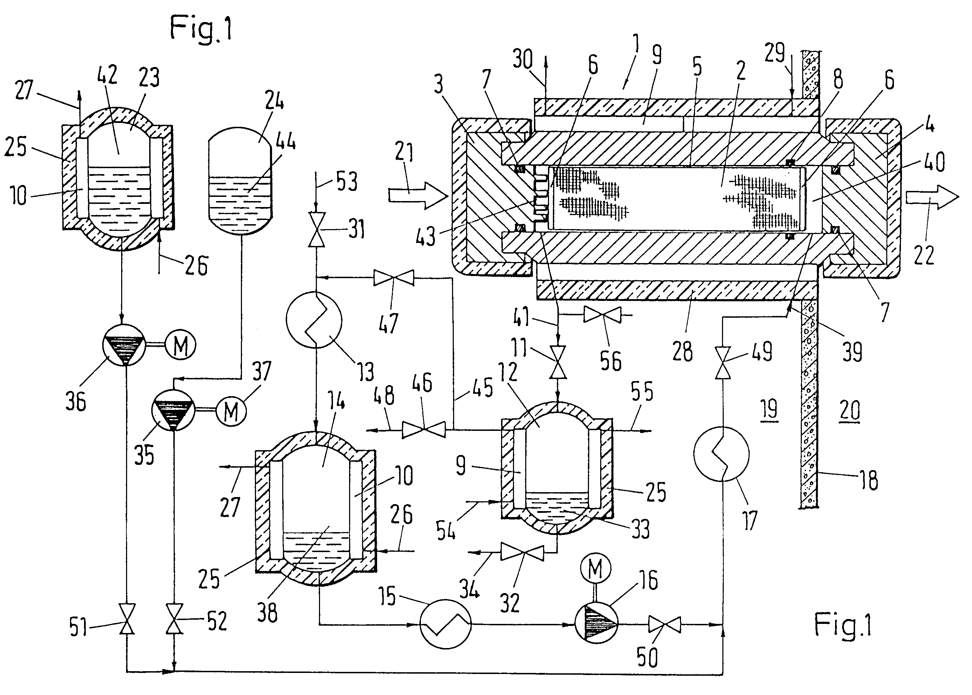

- Figur 1: schematisch den Schnitt durch eine Waschkammer mit eingefülltem Waschgut und die Anordnung der restlichen Elemente zur Waschkammer.

Die Figur zeigt eine Hochdruck-Waschkammer in der ein Waschgut, das aus textilen medizinischen Implantaten besteht, von der Atmosphäre isoliert eingeschlossen ist. Das textile medizinische Implantat wird zur Reduktion der Keime von einem überkritischen oder als flüssiges Fluid ausgebildeten Waschmedium für eine bestimmte Waschzeit durchströmt, wobei das Fluid bei Umgebungsbedingungen aus mindestens einem nicht-toxischen als Hauptwaschmittel wirkenden Gas besteht, dem nach Bedarf weitere Gase und/oder flüssige Lösungsmittel beigemischt werden können. Das in Vorratsbehältern in flüssiger Form gelagerte Waschmedium wird durch Pumpen auf einen so hohen Druck gebracht, dass es nach dem Zusammenmischen die Waschkammer bei der vorgesehenen Waschtemperatur als überkritisches oder flüssiges Fluid durchströmt. Dabei sind Temperatur und Druck des Waschmediums entsprechend einem vorgegebenen Programm regelbar, um den keimmindernden überkritischen Zustand mit seiner Temperatur einer maximal zulässigen Temperatur des textilen medizinischen Implantats anzupassen.

Das Beispiel in Figur 1 zeigt eine Hochdruck-Waschkammer 1, die aus einem mittleren Rohrstück, einem Einfülldeckel 3 und einem Entnahmedeckel 4 besteht. Die Deckel 3, 4 sind über Hochdruckdichtungen 7 gegen das Rohrstück abgedichtet und abnehmbar. Das textile medizinische Implantat 2 wird von einem Anlieferraum 19 in Anlieferrichtung 21 in einer Kartouche 5 bei abgenommenem Einfülldeckel 3 in die Waschkammer 1 eingeschoben, während der Entnahmedeckel 4 geschlossen bleibt. Dabei wird die Mantelfläche der Kartouche 5 über eine Weichdichtung 8 zum mittleren Rohrstück abgedichtet. Mit dem Schliessen des Einfülldeckels 3 liegt die Kartouche 5 auf der Einfüllseite mit ihrem Deckel 6 an Stützrippen 43 des Einfülldeckels 3 an. Die beiden Deckel 6 der Kartouche 5 sind aus Sintermetall und sorgen mit ihren Poren für eine gleichmässige Strömungsverteilung über den querschnitt der Waschkammer 1. Das mittlere Rohrstück der Waschkammer 1 ist mit einem Heizmantel 9 versehen, der von einem Eintritt 29 zu einem Austritt 30 von einem Heizmedium durchflossen wird. Die äussere Oberfläche der Waschkammer 1 ist mit einer Wärmeisolation 28 abgedeckt.

Nach dem Entkeimungs- und Waschvorgang wird der Eintritt 39 des Waschmediums mit Sperrventil 49 und der Austritt 41 mit Ventil 11 geschlossen und es wird über Sperrventil 56 ein Druckausgleich zur Atmosphäre hergestellt. Anschliessend wird der Entnahmedeckel 4 zum Ablieferraum 20 geöffnet, dessen Atmosphäre durch eine Trennwand 18 vom Anlieferraum 19 getrennt ist und der Teil eines konditionierten Reinstraums ist. Die Kartouche 5 mit dem Waschgut 2 wird in Ablieferrichtung 22 entnommen und der Entnahmedeckel 4 wird wieder geschlossen. Das textile medizinische Implantat 2 wird aus der Kartouche 5 entfernt und weiterverarbeitet.

Vor dem eigentlichen Waschen des in der Waschkammer 1 eingeschlossenen Waschguts 2 findet eine Spülung mit Hauptwaschmittel 38, statt um fremde Gase aus der Waschkammer 1 zu verdrängen.

Beim eigentlichen Waschvorgang wird aus einem gekühlten Vorratsbehälter 14 flüssiges Hauptwaschmittel 38 angesogen und in einem Unterkühler 15 weiter unterkühlt, bevor es eine Druckerhöhung in einer Pumpe 16 erfährt. Zwischen Pumpe 16 und dem Eintritt 39 für das Waschmedium 40 wird ein Wärmetauscher 17 durchflossen, mit dem die Temperatur der Waschflüssigkeit 40 auf die richtige Eintrittstemperatur geregelt wird. Der Druck, gegen den die Pumpe 16 fördert, wird durch die Druckverluste bis zum Entspannungsventil 11 am Austritt 41 der Waschkammer 1 und durch das Entspannungsventil selbst bestimmt. Bei künstlichen Bänderimplantaten und bei CO₂ als Hauptwaschmittel 38 sind für das Waschmedium 40 eine Temperatur von 50°C und ein Druck von 150 bar typische Waschdaten.

Mit der Entspannung des beladenen Waschmediums über das Entspannungsventil 11 findet eine drastische Dichteverminderung und somit eine starke Verkleinerung der Löslichkeit statt. Im Waschmedium gelöste Rückstände werden frei. Das zweiphasige Gemisch aus praktisch reinem Waschmittel und ausgefällten Rückständen wird weiter in einen beheizten Abscheidebehälter 12 geführt, der mit einem Heizmantel 9 und mit einem Eintritt 54 und einem Austritt 55 für ein Heizmedium ausgerüstet ist und in welchen die Löslichkeit weiter vermindert und die zwei Phasen voneinander getrennt werden. Das Hauptwaschmittel 38 verlässt den Abscheider als Gas während sich sonstige Rückstände 33 im Sumpf des Abscheiders sammeln und über einen Austritt 34 und ein dazugehöriges Abzugsventil 32 abgezogen werden können. Bei einem geschlossenen Kreislauf sind Nachfüllventil 31 und Sperrventil 46 mit Spülausgang 48 geschlossen, damit das gasförmige Hauptwaschmittel aus dem Abscheidebehälter 12 über einen Kondensator 13 in den Vorratsbehälter 14 zurückgelangt. Bei einem offenen Kreislauf ist das Sperrventil 47 in Rücklaufleitung 45 geschlossen und das Hauptwaschmittel wird über Spülausgang 48 und das offene Sperrventil 46 freigesetzt. Gleichzeitig muss dann auch frisches Hauptwaschmittel 53 über das Nachfüllventil 31 nachgefüllt werden.

Da in diesem Verfahren das Reinigen durch Lösen geschieht und deswegen nur kleine Strömungsgeschwindigkeiten für das Waschmedium 40 notwendig sind, kann bei umweltverträglichen nicht brennbaren Hauptwaschmitteln 38 wie z.B. CO₂ auch ein offenes System sinnvoll sein. Für den reinen Waschvorgang genügt es, das Volumen der Waschkammer 1 weniger als 10 mal pro Stunde durchzusetzen.

Waschmedien, die unter Umgebungsbedingungen nicht toxisch wirken, können im superkritischen Zustand auch bei Temperaturen, welche erheblich unter der Dampfsterilisationstemperatur liegen, keimtötend wirken. Es ist daher ein Ziel der Erfindung mit solchen Medien zu arbeiten, um die Umwelt und das Bedienungspersonal nicht unnötig zu gefährden. Praktisch wird ein superkritischer Zustand des Waschmediums erzeugt, dessen Temperatur nicht höher ist als eine durch das textile medizinische Implantat bestimmte Temperatur, die so gewählt ist, dass sie für die mechanischen Eigenschaften des später im Körper eingesetzten Implantats unbedenklich ist. Aus diesem Grund sind Temperatur und Druck des Waschmediums regelbar, um auch unterhalb einer für das Implantat gefährlichen Temperatur einen superkritischen Zustand zu erzeugen.

Weiterhin hat sich gezeigt, dass bei einer bestimmten Zusammensetzung des Waschmediums die optimale Löslichkeit von in chemischer Hinsicht verschiedenen Schmutzstoffen bei unterschiedlichen Kombinationen von Temperatur und Druck des Waschmediums liegt.

Im Waschprogramm werden daher am Waschmedium 40 Temperatur und Druck auch verändert, um das Lösungsspektrum des Waschmediums zu verbessern.

Eine weitere Vergrösserung des Lösungsspektrums kann unter Umständen durch das Beimischen von flüssigen Lösungsmitteln (Modifier) 44, die aus einem Vorratsbehälter 24 über eine Pumpe 35, welche mit einem Antriebsmotor 37 angetrieben wird, dem Strom des Hauptwaschmittels 38 vor dem Eintritt in den Wärmetauscher 17 zugemischt werden, erreicht werden. Solche bei Umgebungsbedingungen flüssigen Lösungsmittel können z.B. Alkohole wie Aethanol oder Methanol sein. Sie durchlaufen mit dem Hauptwaschmittel 38 die Waschkammer 1 und können im Abscheidebehälter 12 in flüssiger Form abgeschieden werden.

Eine weitere Vergrösserung des Lösungsspektrums kann dadurch entstehen, dass das Hauptwaschmittel 38 ein Gas oder eine Gasmischung ist, deren Konzentration durch Zumischen weiterer Gase 42 gezielt verändert wird. Solche Gase können CO₂, Butan, Propan sein. Die weiteren Gase 42 werden in einem Vorratsbehälter 23 mittels Unterkühlung mit einem Kühlmantel 10, der über Eintritt 26 und Austritt 27 von einem Kühlmedium durchflossen wird, flüssig gehalten. Eine Pumpe 36 fördert die verflüssigten Gase 42 aus dem Vorratsbehälter 23 und speist sie in den Strom des Hauptwaschmittels 38 bevor es in den Wärmetauscher 17 eintritt.

Die meisten textilen medizinischen Implantate werden durch die Temperaturen der Dampfsterilisation geschädigt. Die Temperaturführung der Waschkammer 1 kann in einem Abstand unter der Dampfsterilisationstemperatur gehalten werden und es wird trotzdem ein hoher Grad an Sterilisation am textilen medizinischen Implantat mit dem Reinigungsprozess erreicht.

Da das Waschgut 2 nur einer sehr langsamen Strömung des Waschmediums ausgesetzt ist und ohne weitere Massnahmen eine in sich unveränderte Lage einnimmt kann es bei Geweben mit vielen Berührungspunkten vorteilhaft sein, mechanische Schwingungen auf das Waschgut zu übertragen. Dazu kann die Waschkammer 1 auf einer Rüttelvorrichtung aufgebaut werden oder es können Druckpulsationen mit einem Schwingungsgeber am Waschmedium 40 in der Waschkammer 1 erzeugt werden. Die Vorratsbehälter 14, 23 und der Abscheidebehälter 12 sind mit einer Wärmeisolation 25 versehen.

## Patentansprüche

1. Verwendung eines Waschverfahrens, bei dem ein Waschgut in eine von der Atmosphäre abschliessbare Waschkammer (1) eingebracht wird, welche nach dem Abschliessen von einem als überkritisches oder flüssiges Fluid ausgebildeten Waschmedium (40) für eine bestimmte Waschzeit durchströmt wird, wobei das Fluid bei Umgebungsbedingungen aus mindestens einem als Hauptwaschmittel wirkenden Gas (38) und aus eventuell zusetzbaren flüssigen Lösungsmitteln (44) besteht, um nach Ablauf der Waschzeit einen Druckausgleich zur Atmosphäre herzustellen und das Waschgut (2) zu entnehmen, zum Entkeimen von einem Waschgut, welches aus textilen medizinischen Implantaten besteht.

2. Verwendung des Verfahrens nach Anspruch 1, bei dem das als Hauptwaschmittel wirkende Gas (38, 42) aus CO₂ oder/und Gasen wie Propan, Butan besteht.

3. Verwendung des Verfahrens nach Anspruch 1, bei dem während des Waschvorgangs, während dem die Waschkammer (1) von einem Waschmedium (40) durchströmt wird, der Druck und die Temperatur des in der Waschkammer (1) befindlichen Waschmediums (40) nach einem bestimmten Programm verändert werden, um die Temperatur des überkritischen Zustands einer maximal zulässigen Temperatur des textilen medizinischen Implantats anzupassen.

4. Verwendung des Verfahrens nach einem der Ansprüche 1 bis 3, bei dem während des Waschvorgangs, während dem die Waschkammer von einem Waschmedium (40) durchströmt wird, die Zusammensetzung des Waschmediums (40) nach einem bestimmten Programm verändert wird, um die Keimverminderung des Waschmediums zu steuern.

5. Verwendung des Verfahrens nach einem der Ansprüche 1 bis 4, bei dem dem Hauptwaschmittel (40) als flüssige Lösungsmittel (44) organische Verbindungen wie z.B. Aethanol, Methanol oder andere Alkohole beigemischt werden.

6. Verwendung des Verfahrens nach einem der Ansprüche 1 bis 5, bei dem das textile medizinische Implantat (2) einer gleichmässig verteilten Strömung des Waschmediums (40) ausgesetzt wird.

7. Verwendung des Verfahrens nach einem der Ansprüche 1 bis 6, bei dem der über die Waschzeit in der Waschkammer theoretisch aufintegrierte Volumenstrom des Waschmediums (40), kleiner ist als das 10-fache Volumen der Waschkammer.

8. Verwendung des Verfahrens nach einem der Ansprüche 1 bis 7, bei dem mechanische Schwingungen auf das textile medizinische Implantat (2) übertragen werden, um den Stoffaustausch und damit den Reinigungsvorgang zu beschleunigen.

9. Verwendung des Verfahrens nach einem der Ansprüche 1 bis 8, bei dem das textile medizinische Implantat (2) von einem Anlieferraum (19) nach dem Oeffnen eines Einfülldeckels (3) in die sonst geschlossene Waschkammer (1) eingebracht wird und nach dem Waschvorgang durch Oeffnen eines Entnahmedeckels (4) zu einem Ablieferraum (20) in diesen entladen wird.

10. Verwendung des Verfahrens nach einem der Ansprüche 1 bis 9, bei dem die Temperatur des Waschmediums (40) in der Waschkammer (1) soweit erhöht wird, dass mit der Reduktion der Keime eine Sterilisation des textilen medizinischen Implantats (2) erreicht wird.

11. Reinigungseinrichtung zur Verwendung des Verfahrens nach einem der Ansprüche 1 bis 10, welche aus einer Waschkammer (1) mit einem Einfülldeckel (3), mit einer Heizeinrichtung (9) und mit einer Zuleitung (39) und einer Ablaufleitung (41) für ein Waschmedium (40) besteht, dadurch gekennzeichnet, dass die Waschkammer (1) als Hochdruck-Autoklav ausgeführt ist, dass Druck und Temperatur des Waschmediums (40) regelbar sind und dass das Waschmedium (40), die Waschkammer (1) mit einem eingeschlossenen textilen medizinischen Implantat (2) als überkritisches oder flüssiges Fluid durchströmt, welches bei Umgebungsbedingungen aus mindestens einem als Hauptwaschmittel wirkenden Gas (38) besteht, dem flüssige Lösungsmittel (44) und/oder weitere Gase (42) beigemischt sein können.

12. Reinigungseinrichtung nach Anspruch 11, dadurch gekennzeichnet, dass die Waschkammer (1) einen Einfülldeckel (3) zu einem Anlieferraum (19) und einen Entnahmedeckel (4) zu einem Ablieferraum (20) aufweist, dessen Atmosphäre durch eine Trennwand (18) vom Anlieferraum (19) getrennt ist.

13. Reinigungseinrichtung nach einem der Ansprüche 11 oder 12, dadurch gekennzeichnet, dass das textile medizinische Implantat (2) in der Waschkammer (1) einer Strömung des Waschmediums (40) von der Ablieferseite mit Deckel (4) zur Anlieferseite mit Deckel (3) unterworfen ist und eine gleichmässige Strömungsverteilung über den querschnitt der Waschkammer (1) vorliegt.

14. Reinigungseinrichtung nach Anspruch 13, dadurch gekennzeichnet, dass das textile medizinische Implantat (2) in einer Kartouche (5), deren Mantelfläche über eine Weichdichtung (8) zur Waschkammer (1) abdichtbar ist und deren Deckel (6) porös ausgebildet sind, in die Waschkammer (1) einbringbar ist.

15. Reinigungsvorrichtung nach einem der Ansprüche 11 bis 13, dadurch gekennzeichnet, dass mehrere Vorratsbehälter (23, 24) vorhanden sind, aus denen wahlweise weitere Gase (42) und/oder flüssige Lösungsmittel (44) dem Hauptwaschmittel (38) zumischbar sind.

16. Reinigungsvorrichtung nach einem der Ansprüche 11 bis 13, dadurch gekennzeichnet, dass die Reinigungseinrichtung einen Vorratsbehälter (14) mit Hauptwaschmittel (38) aufweist, welches in die Waschkammer (1) förderbar ist und dass das aus der Waschkammer (1) ausfliessende beladene Waschmedium (40) nach einer Drucksenkung in einem Entspannungsventil (11) und/oder nach einer Temperaturveränderung über einen Abscheidebehälter (12) geführt ist, um Rückstände (33) und eventuelle Zusätze abzuscheiden und um sauberes Hauptwaschmittel (38) über eine Rückführleitung (45) in den Vorratsbehälter (14) zurückzuführen.
